**Europäisches Patentamt**

**European Patent Office**

**Office européen des brevets**

(19)

(11) Publication number: **0 006 223**
**A1**

# EUROPEAN PATENT APPLICATION

(12)

(21) Application number: **79101926.8**

(22) Date of filing: **13.06.79**

(51) Int. Cl.³: **C 07 D 209/28, A 61 K 31/405**

(30) Priority: **13.06.78 US 915105**

(71) Applicant: **Merck & Co., Inc., Patent Department 126 East Lincoln Avenue, Rahway, N.J. 07065 (US)**

(43) Date of publication of application: **09.01.80**
**Bulletin 80/1**

(72) Inventor: **Ryan, James Arthur, 72 Scarlet Oak Drive, Doylestown, Pa. 18901 (US)**

(84) Designated Contracting States: **AT BE CH DE FR GB IT LU NL SE**

(74) Representative: **Abitz, Walter, Dr.-Ing. et al, Abitz, Morf, Gritschneder P.O.Box 860109, D-8000 München 86 (DE)**

(54) Crystalline sodium and potassium indomethacin and their trihydrates, process for preparing and pharmaceutical compositions containing the same.

(57) Sodium and potassium 1-p-chlorobenzoyl-2-methyl-5--methoxy-3-indolyl acetate and their trihydrates are stable crystalline materials prepared by crystallization from water and acetone and are useful in facilitating the preparation of ophthalmic, parenteral and other formulations of indomethacin for treatment of inflammatory conditions.

EP 0 006 223 A1

## TITLE OF THE INVENTION

CRYSTALLINE SODIUM AND POTASSIUM INDOMETHACIN AND
THEIR TRIHYDRATES, PROCESS FOR PREPARING AND PHARMA-
CEUTICAL COMPOSITIONS CONTAINING THE SAME

## BACKGROUND OF THE INVENTION

### Field of the Invention

The present invention is concerned with
crystalline sodium and potassium l-p-chlorobenzoyl-
2-methyl-5-methoxy-3-indolyl acetate and their trihydrates and improved pharmaceutical compositions prepared therefrom.

### Description of the Prior Art

The compound l-p-chlorobenzoyl-2-methyl-
5-methoxy-3-indolyl acetic acid is a potent non-
steriodal anti-inflammatory agent described in U.S.
Patent No. 3,161,654. While the alkali metal,
particularly sodium and potassium salts of the acid
are also mentioned, they are not described as being
stable, crystalline materials, and it is only recited
that the salts may be obtained by treatment of the
free acid with base under mild conditions. As is
shown further below, a stable crystalline material
is obtained only with the application of carefully
controlled conditions to the preparation of the
sodium, potassium and trihydrate salts.

SUMMARY OF THE INVENTION

The treatment of inflammation by chemotherapeutic agents has advanced a great distance in the last three decades, with the development of many new chemotherapeutic agents such as the steriods, followed by a number of non-steriodal agents.  One of these, a potent drug known as indomethacin, is 1-p-chlorobenzoyl-2-methyl-5-methoxy-3-indolyl acetic acid.  This drug may be administered by way of a number of different formulation types, depending upon the particular inflammatory condition which it is desired to treat.  However, most such formulations would be improved, and their preparation facilitated, by the use therein of a stable, crystalline, readily-dissolved salt of indomethacin acid.

In the past, the problem of the insolubility of indomethacin acid has been overcome simply by preparing tablets from fine particles, a suspension formulation, or an organic solvent solution of the indomethacin.  Where a sodium or potassium salt of indomethacin has been required, indomethacin acid has been treated with aqueous alkali and the reaction mixture has simply been lyophilized to a solid.  However, a number of difficulties have been experienced in preparing such lyophilized sodium and potassium indomethacin salts.  Slow and incomplete dissolution of indomethacin even with the aid of a surfactant requires high pH's with resulting degradation caused by hydrolysis cleavage of the 1-p-chlorobenzoyl group.

The problems previously encountered with insoluble, hard-to-dissolve, and lyophilized salts of indomethacin have been overcome by the present invention whereby there is provided readily dissolved, stable, crystalline sodium and potassium indomethacin and their trihydrates. In accordance with the method of the present invention, the sodium or potassium trihydrate salt of indomethacin is first prepared by reacting indomethacin acid with sodium or potassium hydroxide, carbonate, or $C_{1-4}$ alcoholate, for example ethylate, in an aqueous medium. The molar ratio of indomethacin acid to sodium or potassium base is 1:0.90 to 0.99 and the pH of the reaction mixture is from 8.0 to 8.5. The pH of the reaction mixture should not exceed 8.5. The temperature of the reaction mixture is preferably maintained at from 1° to 10°C in order to avoid hydrolysis cleavage of the 1-p-chlorobenzoyl group. The sodium or potassium base as an aqueous solution is added to an aqueous mixture of the indomethacin acid over a period of from 1.5 to 3.0 hours. After addition is complete, the reaction mixture is poured gradually into acetone over a period of from 0.5 to 2.0 hours. The volume of acetone employed should be such that the ratio of acetone volume to the total volume of the aqueous sodium or potassium base solution and aqueous mixture of indomethacin acid together, is from 5:1 to 8:1. The sodium or potassium indomethacin trihydrate thus prepared crystallizes out from the acetone/aqueous mixture and is then separated by filtration and purified by washing. The sodium or potassium indomethacin trihydrate is a readily-dissolved, stable, crystalline material. The crystalline sodium indomethacin trihydrate has been submitted to various analyses with the following results: the Karl Fischer (KF) moisture is 13.3% (theoretical trihydrate moisture is 13.45%);
ultraviolet analysis, with A% in parentheses, is:
318 mμ (147), 261 mμ (381), 220 mμ (476); infrared
analysis shows that probably most of the water is
in the crystal lattice; microscopic analysis reveals a material which is a fluffy powder of crystalline rods and needles in aggregated form and strongly
birefringent; X-ray diffraction analysis shows the
material to be crystalline; and thin layer chromatography indicates a single compound.

The crystalline sodium or potassium indomethacin is prepared, in turn, from the crystalline
sodium or potassium indomethacin trihydrate by drying. This drying is preferably accomplished by
air drying at room temperature for 0.25 to 1.0 hour,
followed by drying in vacuo at room temperature for
1 to 12 hours. The resulting sodium or potassium
indomethacin is a readily-dissolved, stable,
crystalline material. However, it has been observed
that rehydration of the sodium indomethacin to form
sodium indomethacin trihydrate will occur upon exposure to the atmosphere. Ultraviolet analysis of
the sodium indomethacin crystalline material gives
the following results, with the A% observed in
parentheses: 3200 mμ (168); 2625 mμ (393); 2320 mμ
(535). X-ray diffraction analysis has confirmed that
the material is highly crystalline.

The crystalline sodium or potassium indomethacin and sodium or potassium indomethacin trihydrate of the present invention are all useful in
facilitating the preparation of improved ophthalmic,
parenteral and other formulations of indomethacin for
treatment of inflammatory conditions. However, as a practical matter, use of the crystalline sodium or potassium indomethacin trihydrate is preferred since their preparation requires fewer steps than preparation of the anhydrous sodium or potassium indomethacin, which is actually prepared from the trihydrate.

Use of the crystalline sodium and potassium indomethacin salts of the present invention in preparing various formulations provides a number of advantages over formulations prepared from amorphous sodium indomethacin. For example, higher concentrations of indomethacin are obtainable with significantly lower pH's and no degradation of the indomethacin occurs.

The present invention will be better understood from the following examples which illustrate preparation of the crystalline sodium indomethacin and trihydrate, as well as representative pharmaceutical formulations containing them.

## EXAMPLE 1

In a 5 liter flask under nitrogen was placed 1 liter of water and 358 g. (1.0) mole of 1-p-chlorobenzoyl-2-methyl-5-methoxy-3-indolyl acetic acid. The reaction mixture was stirred to a wet solid and was then cooled to 3°-5°C. with wet ice. Over the next 2.25 hours there was added 950 ml. of 1N sodium hydroxide. The pH of the reaction mixture was maintained at 8.2 to 8.5, and not permitted to exceed 8.5. After addition of the sodium hydroxide, the reaction mixture was stirred at 3°-7°C. for 20 minutes. The reaction mixture was then filtered into 11.4 liters of acetone over a period of 1.25 hours, after which the reaction mixture was filtered to remove unreacted 1-p-chlorobenzoyl-2-methyl-5-methoxy-3-indolyl acetic acid. Then an additional 3.8 liters of acetone was added to the reaction mixture and it was stirred at 5°C. for 1.5 hours, after which it was filtered and the packed filter cake washed twice with 500 ml. of cold (5°C.) acetone and once with 150 ml. of cold acetone. The product was dried in vacuo at room temperature overnight. The weight of product recovered was 200g. (57%).

For the purpose of treating arthritic disorders or like conditions which are responsive to anti-inflammatory drugs, the crystalline sodium or potassium indomethacin and trihydrate of the present invention may be administered orally, ophthalmically, topically, parenterally, by inhalation spray or rectally in dosage unit formulations containing conventional non-toxic pharmaceutically acceptable carriers, adjuvants and vehicles. The term parenteral is intended to include subcutaneous injections, intravenous, intramuscular, intrasternal injection or infusion techniques. In addition to the treatment of warm-blooded animals such as mice, rats, horses, dogs, cats, etc., the crystalline salts of the present invention are effective in the treatment of humans.

The pharmaceutical compositions containing the active ingredient may be in a form suitable for oral use, for example, as tablets, troches, lozenges, oily suspensions, emulsions, hard or soft capsules, syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide a pharmaceutically elegant and palatable preparation.

Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, maize starch, or alginic acid; binding agents, for example starch, gelatine or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed.

Formulations for oral use may also be presented as hard gelatine capsules wherein the active ingredient is mixed with an inert solid diluent, for example, calcium carbonate, calcium phosphate or kaolin, or as soft gelatine capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyoxyethylene sorbitan monooleate. The said aqueous suspensions may also contain one or more preservatives, for example ethyl, or n-propyl, p-hydroxy benzoate, one or more coloring agents, one or more flavoring agents, and one or more sweetening agents, such as sucrose or saccharin.

Oily suspension may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an antioxidant such as ascorbin acid.

The pharmaceutical compsoitions of the invention may also be in the form of oil-in-water emulsions. The oily phase may be a vegetable oil, for example olive oil or arachis oils, or a mineral oil, for example liquid paraffin or mixtures of these. Suitable emulsifying agents may be naturally-occurring gums, for example gum acacia or gum tragacanth, naturally-occurring phosphatides, for example soya bean lecithin, and esters or partial esters derived from fatty acids and hesitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, sorbitol or sucrose. Such formulations may also contain a demulcent, a preservative and flavoring and coloring agents. The pharmaceutical compositions may be in the form of a sterile injectable preparation, for example as a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1, 3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The crystalline salts of the invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies solutions or suspensions, etc., containing the anti-inflammatory agents are employed.

The amount of active ingredient which may be combined with the carrier materials to produce a single dosage form will vary depending upon the host treated and the particular mode of administration. For example, a formulation intended for the oral administration of humans may contain 5 mg. to 5 gm. of active agent compounded with an appropriate and convenient amount of carrier material which may vary from about 5 to about 95 percent of the total composition. Dosage unit forms will generally contain between from about 25 mg. to about 500 mg. of active ingredient.

The following examples are illustrative of typical pharmaceutical compositions of the present invention and their preparation.

### EXAMPLE 2

The following ingredients are employed in preparing a sterile lyophilized indomethacin formulation for I.M. injection, containing 50 mg. of active ingredient per vial:

| Ingredient | Amount |
|---|---|
| indomethacin sodium .$3H_2O$ | 62.4 mg. (equivalent to 50 mg. indomethacin plus 3% overage) |
| Emulphor EL-620 (polyoxy-ethylated vegetable oil) | 3.13 mg. |
| mannitol | 78.1 mg. |
| water for injection USP q.s. | 2.0 ml. |

The non-sterile formulation is prepared as follows: (a) in a suitable glass container place the water for injection; (b) add and dissolve with agitation the Emulphor EL-620 and indomethacin sodium .3H$_2$O; (c) add and dissolve the mannitol; (d) bring up the volume of solution with water for injection q.s.; and (e) mix thoroughly to effect a uniform solution.

Sterilization of the formulation as prepared above is carried out as follows: (a) sterilize the bulk solution with a sterile 0.22 micron sterilizing filter with pre-filter; (b) collect the sterile filtrate in a suitable sterile glass vessel equipped for product subdivision; (c) aseptically subdivide the bulk solution through an in-line or final filter system, filling 2.0 ml. per vial using a suitable sterile filter; (d) use any Type I, 13 mm. molded vial of 3 ml. to 5 ml. capacity; (e) aseptically apply suitable butyl 815 sterile fluted lyo stoppers but do not completely insert; and (f) aseptically transfer the filled vials to a sterile lyo chamber and freeze to at least -30°C. for 2 hrs. or more.

Lyophilization of the sterilized formulation as prepared above is carried out as follows: (a) when the product has been frozen (-30°C.) for at least 2 hrs., apply vacuum to the chamber, (b) when the vacuum is below 100 microns for at least 2 hrs., set the shelf temperature at +25°C.; (c) when the product temperature reaches 25°C. (usually 24 hrs.), raise the shelf temperature to +30° - 40°C. until the product temperature reaches +30°C.; (d) maintain the product temperature at +30°C. for 2 hrs.; (e) stopper the vials internally and remove from the evacuated lyo chamber; and (f) apply any suitable aluminum seal.

-12-                    16203

### EXAMPLE 3

A sterile, lyophilized indomethacin formulation for I.V. injection is prepared in accordance with the procedures described in Example 2 above, except that water and indomethacin sodium $3H_2O$ are the only two ingredients employed, the Emulphor EL-620 and mannitol being omitted.

WHAT IS CLAIMED IS:

1.  A readily dissolved, stable, crystalline salt of 1-p-chlorobenzoyl-2-methyl-5-methoxy-3-indolyl acetic acid selected from the sodium, potassium, sodium trihydrate, or potassium trihydrate salts thereof.

2.  Readily dissolved, stable, crystalline sodium 1-p-chlorobenzoyl-2-methyl-5-methoxy-3-indolyl acetate trihydrate.

3.  A pharmaceutical composition for use in treating inflammatory conditions comprising as active ingredient an effective amount of a readily dissolved, stable, crystalline salt of 1-p-chlorobenzoyl-2-methyl-5-methoxy-3-indolyl acetic acid selected from the sodium, potassium, sodium trihydrate, or potassium trihydrate salts thereof, and a non-toxic, pharmaceutically acceptable carrier.

4.  A pharmaceutical composition for use in treating inflammatory conditions comprising as active ingredient an effective amount of readily dissolved, stable, crystalline sodium 1-p-chlorobenzoyl-2-methyl-5-methoxy-3-indolyl acetate trihydrate and a non-toxic, pharmaceutically acceptable carrier.

5.  A method of preparing a readily dissolved, stable, crystalline salt of 1-p-chloropbenzoyl-2-methyl-5-methoxy-3-indolyl acetic acid selected from the sodium and potassium salts thereof, comprising the steps of

-14-          16203

(a)    reacting 1-p-chlorobenzoyl-2-methyl-5-methoxy-3-indolylacetic acid with sodium or potassium hydroxide, carbonate, hydrogen carbonate, or $C_{1-4}$ alcoholate in an aqueous medium at a pH of from 8.0 to 8.5;

(b)    pouring the reaction mixture of step (a) above into acetone;

(c)    recovering the sodium or potassium 1-p-chlorobenzoyl-2-methyl-5-methoxy-3-indolyl acetate trihydrate which crystallizes out from the reaction mixture of step (b); and

(d)    drying the trihydrate recovered in step (c) to form the sodium or potassium 1-p-chlorobenzoyl-2-methyl-5-methoxy-3-indolyl acetate.

6.    The method of Claim 5 wherein the molar ratio of 1-p-chlorobenzoyl-2-methyl-5-methoxy-3-indolyl-acetic acid to sodium or potassium base is 1:0.90 to 0.99.

7.    The method of Claim 5 wherein the temperature of the reaction mixture in Steps (a) and (b) is from 1° to 10°C.

8.    A method of preparing a readily dissolved, stable, crystalline salt of 1-p-chlorobenzoyl-2-methyl-5-methoxy-3-indolyl acetic acid selected from the sodium trihydrate and potassium trihydrate salts thereof, comprising the steps of

(a)    reacting 1-p-chlorobenzoyl-2-methyl-5-methoxy-3-indolylacetic acid with sodium or potassium hydroxide, carbonate, hydrogen carbonate, or $C_{1-4}$ alcoholate in an aqueous medium at a pH of from 8.0 to 8.5;

(b)  pouring the reaction mixture of step (a) above into acetone; and

(c)  recovering the sodium or potassium 1-p-chlorobenzoyl-2-methyl-5-methoxy-3-indolyl acetate trihydrate which crystallizes out from the reaction mixture of step (b).

9.  The method of Claim 8 wherein the molar ratio of 1-p-chlorobenzoyl-2-methyl-5-methoxy-3-indolyl-acetic acid to sodium or potassium base is 1:0.90 to 0.99.

10. The method of Claim 8 wherein the temperature of the reaction in Steps (a) and (b) is from 1° to 10°C.

## DOCUMENTS CONSIDERED TO BE RELEVANT

| Category | Citation of document with indication, where appropriate, of relevant passages | Relevant to claim |
|---|---|---|
| X | DE - A - 1 770 157 (SUMITOMO CHEMICAL)<br>* claim 6; examples 10 to 12, 40 * | 1,3,5 |
| | US - A - 3 845 210 (N. SATO et al.)<br>* claims 1,2 * | 3,4 |
| A | DE - B - 1 232 150 (MERCK)<br>* column 3, lines 1 to 6 * | |
| D,A | US - A - 3 161 654 (TSUNG-YING SHEN)<br>* column 2, lines 63 to 69 * | |

**CLASSIFICATION OF THE APPLICATION (Int. Cl.3)**

C 07 D 209/28
A 61 K 31/405

**TECHNICAL FIELDS SEARCHED (Int.Cl.3)**

A 61 K 31/405
C 07 D 209/26
C 07 D 209/28

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

| | The present search report has been drawn up for all claims | | |
|---|---|---|---|
| Place of search<br>Berlin | Date of completion of the search<br>06-09-1979 | Examiner<br>FROELICH | |

EPO Form 1503.1  06.78